# EUROPEAN PATENT APPLICATION

(11) **EP 4 729 173 A1**
(43) Date of publication of application: **22.04.2026**
(21) Application number: 25306752.4
(22) Date of filing: 21.10.2025
(51) Int. Cl.: B01J 23/889, B01J 37/08, C07C 31/20, B01J 35/40, B01J 37/18

(54) **PROCESS FOR CONVERTING SUGARS TO GLYCOLS**

(30) Priority: 21.10.2024 EP 24306759
(71) Applicant: Technip Energies France, 92741 Nanterre Cedex (FR)
(72) Inventor: Lepore, Andrew, 92741 Nanterre Cedex (FR); Silverman, Roy, 92741 Nanterre Cedex (FR); Chi, CM, 92741 Nanterre Cedex (FR)
(74) Representative: Edson, Russell Gregory

(57) **Abstract**

Glycols may be produced from sugars using a nickel-rhenium catalyst that includes an active nickel-rhenium mixed metal oxide or alloy on the alpha-alumina particles. The nickel-rhenium catalyst may be produced by impregnating alpha-alumina particles with catalyst precursors including a nickel-containing salt and a rhenium-containing salt to produce precursor-impregnated alpha-alumina particles. The precursor-impregnated alpha-alumina particles may then be dried in a fluidized bed and then calcined, reduced, and passivated to produce a nickel-rhenium catalyst.

## Description

### Technical Field

The present disclosure relates generally to producing glycols. More specifically, but not by way of limitation, this disclosure relates to the production of glycols from sugars using nickel-rhenium catalyst.

### Background

Glycols are used in the production of a wide range of products, including plastics, antifreeze, and solvents. Conventionally, ethylene is converted to ethylene oxide then hydrated to ethylene glycol. Alternate starting materials like sugars have been investigated for producing glycols. However, these methods may often be inefficient and not competitive with ethylene-based routes.

### Summary

One or more embodiments include a method comprising: impregnating alpha-alumina particles with catalyst precursors comprising a nickel-containing salt and a rhenium-containing salt to produce precursor-impregnated alpha-alumina particles; drying of the precursor-impregnated alpha-alumina particles in a fluidized bed at a first temperature; and calcining then reducing then passivating the precursor-impregnated alpha-alumina particles in the fluidized bed to produce a nickel-rhenium catalyst comprising a nickel-rhenium mixed metal oxide or alloy on the alpha-alumina particles.

One or more embodiments include the method of any previous paragraph, wherein the nickel-rhenium catalyst has a total weight ratio of nickel to rhenium ranging from 2:1 to 10:1, optionally ranging from 3.5:1 to 4:1.

One or more embodiments include the method of any previous paragraph, wherein the nickel-rhenium catalyst comprises 1 wt% to 99 wt% of the nickel-rhenium mixed metal oxide or alloy, based on a total weight of nickel and rhenium.

One or more embodiments include the method of any previous paragraph, wherein the nickel-rhenium catalyst has a weight ratio of alumina to nickel and rhenium, cumulatively, ranging from 50:1 to 99.5:1.

One or more embodiments include the method of any previous paragraph, wherein the alpha-alumina particles has at least 90 wt% alpha-alumina.

One or more embodiments include the method of any previous paragraph, wherein impregnating of the alpha-alumina particles comprises contacting the alpha-alumina particles and a precursor solution that comprises the catalyst precursors and water. Optionally, the method may further comprise continuously agitating the alpha-alumina particles while contacting the alpha-alumina particles with the precursor solution.

One or more embodiments include the method of any previous paragraph further comprising: drying the precursor-impregnated alpha-alumina particles at a second temperature before drying at the first temperature, wherein the first temperature is greater than the second temperature. Optionally, the drying at the second temperature is not in a fluidized bed.

One or more embodiments include the method of any previous paragraph, wherein the reducing step is a two-stage reducing step, wherein a first stage is performed at a temperature ranging from 200°C to 300°C and for a time ranging from 3 hours to 12 hours, and wherein a second stage is performed at a temperature ranging from 275°C to 400°C and for a time ranging from 12 hours to 36 hours.

One or more embodiments include a method comprising: producing a reaction mixture comprising a sugar, a nickel-rhenium catalyst, a tungsten co-catalyst, and a solvent to produce a reaction mixture, wherein the nickel-rhenium catalyst comprises a nickel-rhenium mixed metal oxide or alloy on alpha-alumina particles; and contacting the reaction mixture and hydrogen at conditions suitable for hydrogenolysis of the sugar to glycol.

One or more embodiments include the method of any previous paragraph, wherein the nickel-rhenium catalyst has a total weight ratio of nickel to rhenium ranging from 2:1 to 10:1, optionally ranging from 3.5:1 to 4:1.

One or more embodiments include the method of any previous paragraph, wherein the nickel-rhenium catalyst comprises 1 wt% to 99 wt% of the nickel-rhenium mixed metal oxide or alloy, based on a total weight of nickel and rhenium.

One or more embodiments include the method of any previous paragraph, wherein the nickel-rhenium catalyst has a weight ratio of alumina to nickel and rhenium, cumulatively, ranging from 50:1 to 99.5:1.

One or more embodiments include the method of any previous paragraph, wherein the alpha-alumina particles has at least 90 wt% alpha-alumina.

One or more embodiments include the method of any previous paragraph, wherein the reaction mixture comprises 10 wt% to 60 wt% of the sugar.

One or more embodiments include the method of any previous paragraph, wherein the conditions suitable for hydrogenolysis comprise a hydrogen pressure ranging from 700 psia to 1800 psia.

One or more embodiments include the method of any previous paragraph, wherein the conditions suitable for hydrogenolysis comprise a temperature ranging from 160°C to 280°C.

One or more embodiments include the method of any previous paragraph, wherein the glycol comprises ethylene glycol, propylene glycol, or a combination thereof.

One or more embodiments include the method of any previous paragraph further comprising: separating the glycol from other components of the reaction mixture by distillation to produce a glycol product.

One or more embodiments include the method of any previous paragraph, wherein the solvent comprises water and optionally a co-solvent.

One or more embodiments include a nickel-rhenium catalyst comprising a nickel-rhenium mixed metal oxide or alloy impregnated on alpha-alumina particles.

One or more embodiments include the nickel-rhenium catalyst of any previous paragraph, wherein the nickel-rhenium catalyst has a total weight ratio of nickel to rhenium ranging from 2:1 to 10:1.

One or more embodiments include the nickel-rhenium catalyst of any previous paragraph, wherein the nickel-rhenium catalyst comprises 1 wt% to 99 wt% of the nickel-rhenium mixed metal oxide or alloy, based on a total weight of nickel and rhenium.

One or more embodiments include the nickel-rhenium catalyst of any previous paragraph, wherein the nickel-rhenium catalyst has a weight ratio of alumina to nickel and rhenium, cumulatively, ranging from 50:1 to 99.5:1.

One or more embodiments include the nickel-rhenium catalyst of any previous paragraph, wherein the alpha-alumina particles has at least 90 wt% alpha-alumina.

### Brief Description of the Drawings

FIG. 1 illustrates a nonlimiting example of a method of the present disclosure for producing a catalyst suitable for converting sugars to glycols.
FIG. 2 illustrates a nonlimiting example of a method of the present disclosure for converting sugars to glycols.
FIG. 3 is the scanning electron microscopy image of a nickel-rhenium catalyst according to at least one embodiment of the present disclosure.
FIG. 4 illustrates an energy dispersive x-ray spectroscopy plot (counts per second per electron volt) showing the elemental composition of the nickel-rhenium catalyst.

### Detailed Description

The present disclosure relates to methods and catalysts for producing glycols from sugars using a nickel-rhenium catalyst where at least a portion of the nickel and rhenium may be present in a nickel-rhenium mixed metal oxide or alloy.

The methods of the present disclosure convert sugars to glycols in an aqueous-based medium, so the catalyst is continuously exposed to water and high temperatures. Compared to rhenium metal, a nickel-rhenium bimetallic compound may be less susceptible to corrosion. Corrosion may deactivate the catalyst over time and cause a decline in conversion selectivity and overall yield, which may lead to short lifetimes for the catalyst. Advantageously, catalysts of the present disclosure from nickel-rhenium mixed metal oxide or alloy may selectively convert sugars to glycols with less byproducts and higher yields while also having a longer lifetime.

It is noted that the figures show merely preferred embodiments according to the invention, and that the figures are provided by way of examples only and should be understood as such. In the figures, the same or similar reference signs or numbers refer to equal or corresponding parts.

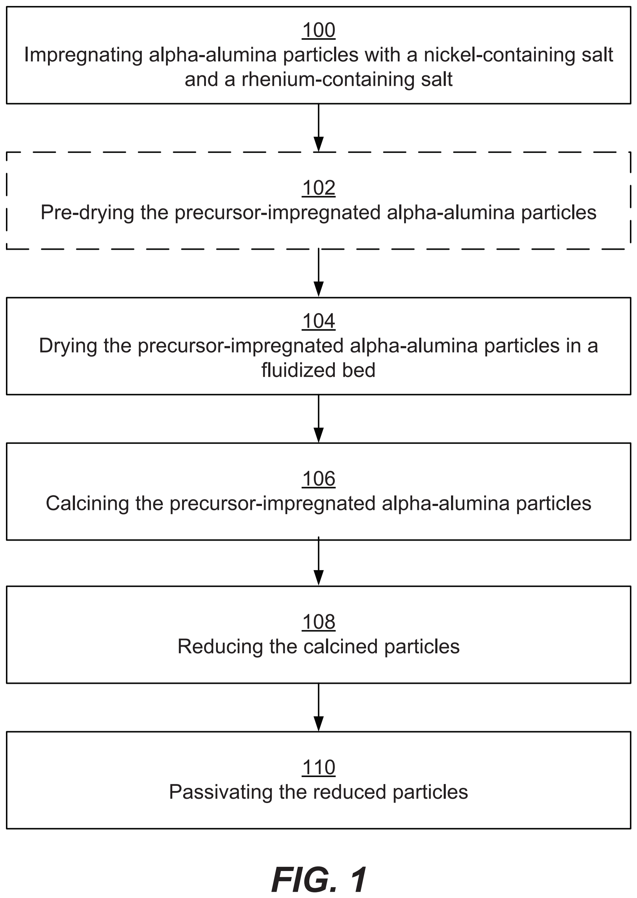

FIG. 1 illustrates a nonlimiting example of a method of the present disclosure for producing a catalyst suitable for converting sugars to glycols. A first step 100 of the method may include impregnating alpha-alumina particles with catalyst precursors that include a nickel-containing salt and a rhenium-containing salt. Impregnation may include contacting the alpha-alumina particles with a precursor solution (e.g., an aqueous-based solution) containing the nickel-containing salt and the rhenium-containing salt.

The alpha-alumina particles and the precursor solution may be contacted using any suitable method that allows the catalyst precursors access to at least the surface of the alpha-alumina particles. In some instances, the alpha-alumina particles may be agitated (e.g., stirred, mixed in a drum mixer, shaken with an agitator, or the like) for at least a portion of time while the precursor solution is applied to the alpha-alumina particles. The rate and manner of addition should be selected to mitigate the formation of alpha-alumina particle agglomerates. Said agglomerates may reduce the homogeneity of distribution of the catalyst precursors throughout the alpha-alumina particles. The agitation also assists with improving the homogeneity of distribution of the catalyst precursors. In some instances, agitation may be continuous while and, optionally, for an amount of time after applying the catalyst precursors. For example, the precursor solution may be added dropwise or sprayed onto the alpha-alumina particles. In some instances, the precursor solution may be added dropwise with 0.1 seconds to 3 seconds (or any other suitable time) between drops with agitation to distribute the drops to more of the alpha-alumina particles. In some instances, a mist of precursor solution may be applied to the alpha-alumina particles periodically over time while continuously agitating the alpha-alumina particles.

Optionally, the alpha-alumina particles may be dried before impregnation. This may remove at least some of the water on the surface of the alpha-alumina particles, thereby facilitating impregnation of more of the catalyst precursors onto the surface and into the porous space of the alpha-alumina particles. Drying may be for a time ranging from 1 hour to 24 hours (e.g., 1 hour to 10 hours, 6 hours to 18 hours, or 12 hours to 24 hours). Drying the alpha-alumina particles may be at any temperature that does not adversely affect the structure of the alpha-alumina particles. For example, the drying temperature may be 60°C or greater (e.g., 60°C to 500°C, 60°C to 400°C, 60°C to 300°C, 60°C to 200°C, 60°C to 150°C, 60°C to 85°C, 75°C to 105°C, 95°C to 120°C, or 105°C to 150°C).

The nickel-containing salt and the rhenium-containing salt may be included in the precursor solution at a suitable concentration and relative ratio to achieve a desired amount of nickel and rhenium in the final catalyst. Such amounts of nickel and rhenium in the final catalyst are provided further herein. A total concentration of catalyst precursors in the precursor solution may range from 0.5 wt% to 10 wt% (e.g., 0.5 wt% to 5 wt%, 3 wt% to 8 wt%, or 5 wt% to 10 wt%).

The precursor solution may include water and, optionally, a solvent (e.g., methanol, ethanol, acetone, the like, and any combination thereof).

The precursor solution may contain one or more nickel-containing salts. A nickel-containing salt may contain nickel (II) or nickel (III) with a counterion selected from nitrate, nitrite, formate, acetate, and the like. The nickel-containing salt may be in a fully or partially hydrated form. An example nickel-containing salt may be nickel (II) nitrate hexahydrate.

The precursor solution may contain one or more rhenium-containing salts (e.g., sodium perrhenate, ammonium perrhenate, or a combination thereof).

The alpha-alumina particles may contain at least 90 wt% (e.g., at least 95 wt%, at least 98 wt%, at least 99 wt%, at least 99.5 wt%, or 100 wt%) alpha-alumina. While other phases of alumina could be present in the support material. The presence of less stable phases of alumina could degrade under reaction conditions.

The average diameter, based on number of particles, may range from 0.1 µm to 1000 µm (e.g., 0.1 µm to 20 µm, 5 µm to 30 µm, 20 µm to 60 µm, 50 µm to 150 µm, 100 µm to 250 µm, 200 µm to 600 µm, or 500 µm to 1000 µm).

The impregnating step 100 may produce precursor-impregnated alpha-alumina particles. Then, the method may optionally include step 102, which includes pre-drying the precursor-impregnated alpha-alumina particles. In some instances, after impregnation, there may be agglomerates of the precursor-impregnated alpha-alumina particles. The pre-drying may remove at least some of the water and other volatile compounds from the precursor-impregnated alpha-alumina particles, which may allow for a more efficient, higher-temperature drying in a later step.

The pre-drying may be at a temperature ranging from 60°C to 110°C (e.g., 60°C to 90°C, 70°C to 100°C, or 80°C to 110°C).

The pre-drying may be performed in an oven at ambient pressure, in a vacuum oven, or in a furnace. Using a vacuum oven may further facilitate removal of at least some of the water and other volatile compounds. In some instances, a furnace may be a vertical furnace where gas (e.g., air, nitrogen, the like, or any combination thereof) is flown through the precursor-impregnated alpha-alumina particles therein. In some instances, the precursor-impregnated alpha-alumina particles in the vertical furnace may be agitated (e.g., using a vibration motor) to reduce slug formation and gas pressure build up.

The pre-drying may be for a time ranging from 1 hour to 24 hours (e.g., 1 hour to 10 hours, 6 hours to 18 hours, or 12 hours to 24 hours).

The method may further include step 104, which includes drying the precursor-impregnated alpha-alumina particles in a fluidized bed. To achieve a fluidized bed, the precursor-impregnated alpha-alumina particles should be individual particles and/or small agglomerates thereof. Accordingly, if a significant amount of water is used in step 100, step 102 may advantageously remove a sufficient amount of water to achieve a desired powder of the precursor-impregnated alpha-alumina particles for step 104.

The precursor-impregnated alpha-alumina particles may be dried in step 104 at a temperature ranging from 80°C to 150°C (e.g., 80°C to 120°C, 100°C to 130°C, or 120°C to 150°C). The temperature of drying in step 104 may be higher than the temperature of the pre-drying in step 102. In some instances, the temperature of drying in step 104 may be at least 10°C higher (e.g., at least 20°C higher, 10°C to 30°C higher, or 15°C to 25°C higher) than the temperature of the pre-drying in step 102.

The precursor-impregnated alpha-alumina particles may be dried as a fluidized bed for a time ranging from 30 minutes to 10 hours or longer (e.g., 30 minutes to 3 hours, 1 hour to 5 hours, or 2 hours to 10 hours). If step 102 is performed, the drying time in step 104 maybe shorter.

Examples of gases used in step 102 and/or step 104 may include, but are not limited to, air, nitrogen, argon, the like, and any combination thereof.

Then, in step 104, the method may include calcining the precursor-impregnated alpha-alumina particles.

The precursor-impregnated alpha-alumina particles may be calcined for a time ranging from 6 hours to 24 hours or longer (e.g., 6 hours to 12 hours, 8 hours to 16 hours, or 12 hours to 24 hours).

The precursor-impregnated alpha-alumina particles may be calcined at a temperature range from 400°C to 600°C (e.g., 400°C to 500°C, 450°C to 550°C, or 500°C to 600°C). In some instances, the calcining may be performed immediately after the drying step 104, where the temperature is increased from the drying temperature to the calcining temperature. In some instance, a heating rate from the drying temperature to the calcining temperature may range from 0.5 °C/minute to 10 °C/minute (e.g., 0.5 °C/minute to 3 °C/minute, 1 °C/minute to 5 °C/minute, or 4 °C/minute to 10 °C/minute).

Examples of gases used in step 106 may include, but are not limited to, air, nitrogen, argon, the like, and any combination thereof. In some instances, a mixture of nitrogen and air may be used, for example, in a volume ratio range from 95:5 to 50:50 (e.g., 95:5 to 65:35, 95:5 to 80:20, or 90:10 to 80:20).

After step 106, the method may include reducing the calcined particles in step 108. Generally, reduction occurs at a lower temperature than calcining. If performing both in the same apparatus, the temperature may be cooled to below a reducing temperature. The rate of reduction in temperature may range from 1 °C/minute to 60 °C/minute (e.g., 1 °C/minute to 15 °C/minute, 10 °C/minute to 40 °C/minute, or 30 °C/minute to 60 °C/minute).

Before, during, or after reaching the reducing temperature, the gas flowing between the calcined particles may be changed, if needed, and maintained for a sufficient time to sufficiently remove oxygen gas.

Then, in step 108, the method may include reducing the calcined particles. The gas flow may be changed to hydrogen, optionally diluted with an inert gas, for example, 1 vol% hydrogen in nitrogen, 10 vol% hydrogen in nitrogen, or 100 vol% hydrogen. The temperature may be increased before, during, or after the hydrogen flow begins. The reducing temperature may range from 200°C to 400°C (e.g., 200°C to 275°C, 250°C to 325°C, or 300°C to 400°C). The rate of temperature increase may range from 0.5 °C/minute to 10 °C/minute (e.g., 0.5 °C/minute to 3 °C/minute, 1 °C/minute to 5 °C/minute, or 4 °C/minute to 10 °C/minute).

Reducing may be performed for a time ranging from 3 hours to 36 hours or longer (e.g., 3 hours to 10 hours, 8 hours to 24 hours, or 18 hours to 36 hours).

In some instances, step 108 may be a two-stage reduction with a first, lower-temperature stage and a second, higher-temperature stage.

The first stage may be at a first reducing temperature that may be from 200°C to 300°C (e.g., 200°C to 250°C, 225°C to 275°C, or 250°C to 300°C) and for a time that may range from 3 hours to 12 hours or longer (e.g., 3 hours to 8 hours, 5 hours to 10 hours, or 8 hours to 12 hours).

The second stage may be at a first reducing temperature that may be from 275°C to 400°C (e.g., 275°C to 350°C, 300°C to 375°C, or 350°C to 400°C) and for a time that may range from 12 hours to 36 hours or longer (e.g., 12 hours to 20 hours, 18 hours to 30 hours, or 24 hours to 36 hours).

The rate of temperature increase from the first stage to the second stage may range from 0.5 °C/minute to 10 °C/minute (e.g., 0.5 °C/minute to 3 °C/minute, 1 °C/minute to 5 °C/minute, or 4 °C/minute to 10 °C/minute).

After the reducing in step 108, the method may include passivating the reduced particles in step 110. The temperature of the reduced particles should be reduced to a passivation temperature before introduction of oxygen. A nitrogen purge may occur to transition the composition of the gas from hydrogen to an oxygen-containing gas. The passivation may occur in an oxygen-containing gas (e.g., oxygen, air, and the like including dilutions of the foregoing with nitrogen).

The passivating temperature may range from room temperature to 100°C (e.g., room temperature to 75°C, room temperature to 60°C, or room temperature to 35°C).

Passivating may occur for a time that may range from 3 hours to 24 hours or longer (e.g., 3 hours to 12 hours, 6 hours to 18 hours, or 12 hours to 24 hours).

Advantageously, steps 102-110 may each be performed with a gas up-flow, which may result in a fluidized bed of the particles. Accordingly, each of these steps may be performed in the same apparatus (e.g., a vertical tube furnace or larger reactor).

One skilled in the art will recognize that there may be intervening steps between the steps illustrated in FIG. 1.

After passivation, the particles may be nickel-rhenium catalysts of the present disclosure.

The nickel-rhenium catalysts of the present disclosure may have a total weight ratio of nickel to rhenium (based on the element present, whether present as a metal, metal oxide, in an alloy, or in any other form) ranging from 2:1 to 10:1 (e.g., 2:1 to 5:1, 3:1 to 6:1, or 5:1 to 10:1).

The nickel-rhenium catalysts of the present disclosure may have a weight ratio of alumina to nickel and rhenium cumulatively (based on each element present, whether present as a metal, in an alloy, or in any other form) ranging from 50:1 to 99.5:1 (e.g., 50:1 to 80:1, 65:1 to 90:1, 80:1 to 95:1, 90:1 to 95:1, 92:1 to 97:1, or 95:1 to 99.5:1).

At least a portion of the nickel and rhenium in the nickel-rhenium catalyst may be present as a nickel-rhenium alloy. In some instances, the amount of nickel-rhenium alloy based on a total weight of nickel and rhenium (whether present as a metal, in an alloy, or in any other form) in the nickel-rhenium catalysts may range from 1 wt% to 99 wt% (e.g., 1 wt% to 25 wt%, 20 wt% to 40 wt%, 35 wt% to 55 wt%, 50 wt% to 70 wt%, 65 wt% to 85 wt%, or 80 wt% to 99 wt%).

At least a portion of the nickel and rhenium in the nickel-rhenium catalyst may be present as separate nickel and rhenium phases.

At least a portion of the nickel and rhenium in the nickel-rhenium catalyst may be present as separate or mixed nickel and rhenium oxide phases.

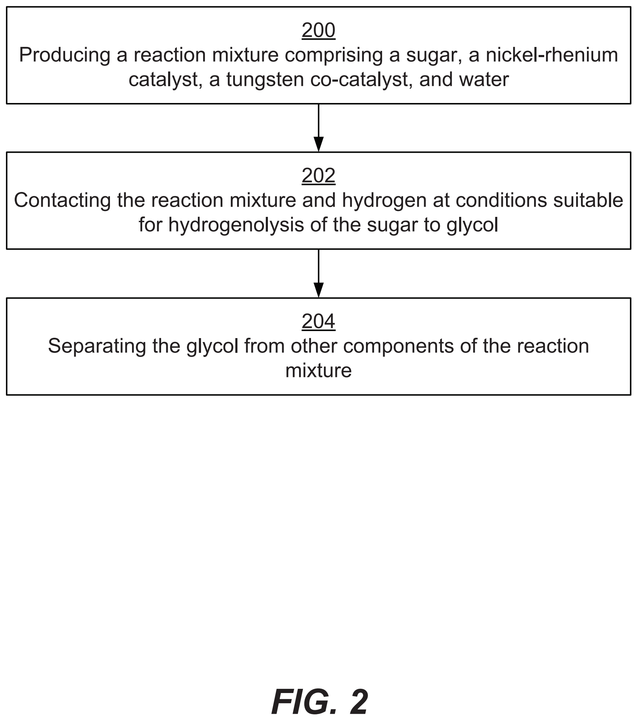

The nickel-rhenium catalysts of the present disclosure may be useful for converting sugars to glycols. FIG. 2 illustrates a nonlimiting example of a method of the present disclosure for converting sugars to glycols. A first step 200 of the method may include producing a reaction mixture comprising sugar, nickel-rhenium catalyst, tungsten co-catalyst, and solvent. The solvent may include water, a co-solvent, such as, methanol, ethanol, propanol, the like, and any combination thereof, or optionally a mixture of water and co-solvent.

One or more sugars may be present in the reaction mixture. Examples of sugars may include, but are not limited to, glucose, fructose, galactose, maltose, lactose, sucrose, allose, altrose, mannose, gulose, idose, talose, arabinose, lyxose, ribose, xylose, ribulose, xylulose, deoxyribose, the like, and any combination thereof.

The sugar may be present in the reaction mixture at a concentration ranging from 10 wt% to 60 wt% (e.g., 10 wt% to 30 wt%, 20 wt% to 40 wt%, or 30 wt% to 60 wt%), based on a total weight of the reaction mixture.

The catalyst load may be at 0.1 g catalyst/1 mL solvent or less (e.g., 0.01 g catalyst/1 mL solvent or less, 0.001 g catalyst/1 mL solvent or 0.01 g catalyst/1 mL solvent, or 0.001 g catalyst/1 mL solvent to 0.1 g catalyst/1 mL solvent).

The tungsten co-catalyst may be in the form of particles having an average diameter, based on number of particles, ranging from 5 µm to 100 µm (e.g., 5 µm to 30 µm, 20 µm to 60 µm, or 50 µm to 100 µm). The tungsten co-catalyst particles may be present in the reaction mixture at a concentration of 0.05 g catalyst/1 mL solvent or less (e.g., 0.01 g catalyst/1 mL solvent or less, 0.0001 g catalyst/1 mL solvent or 0.05 g catalyst/1 mL solvent, or 0.0001 g catalyst/1 mL solvent to 0.01 g catalyst/1 mL solvent).

Alternatively, the tungsten co-catalyst may be a tungsten salt dissolved in the reaction mixture. The tungsten salt concentration may range from 100 ppm to 10,000 ppm in the reaction mixture (e.g., 100 ppm to 2500 ppm, 1500 ppm to 5000 ppm, 2500 ppm to 7500 ppm, or 5000 ppm to 10,000 ppm).

In some examples, the tungsten co-catalyst salt may be present at a concentration less than 3500 ppm. In some further examples, the tungsten co-catalyst salt may be present at a concentration from 100 ppm to 2500 ppm.

The tungsten co-catalyst particles and the tungsten co-catalyst salt may be used in combination.

As second step 202 of the method may include contacting the reaction mixture and hydrogen at conditions suitable for hydrogenolysis of the sugar to glycol, which may occur in a batch reactor or a flow reactor.

Conditions for hydrogenolysis of sugar may include a hydrogen pressure ranging from 700 psia (pounds per square inch absolute) to 1800 psia (e.g., 700 psia to 1200 psia, 900 psia to 1500 psia, or 1200 psia to 1800 psia)

Conditions for hydrogenolysis of sugar may include a temperature ranging from 160°C to 280°C (e.g., 160°C to 220°C, 200°C to 250°C, or 220°C to 280°C).

In a batch reactor, the hydrogenolysis reaction may be for a time ranging from 30 minutes to 10 hours (e.g., 30 minutes to 3 hours, 2 hours to 6 hours, or 5 hours to 10 hours).

The hydrogenolysis reaction may have a percent conversion to glycol of 50% or greater (e.g., 50% or greater, 60% or greater, or 70% or greater).

Byproducts of the reaction may include other polyols like 1,2-propanediol and glycerol.

The method may further include separating the glycol from other components of the reaction mixture in step 204. Examples of separation methods may include, but are not limited to, distillation including multi-effect distillation, heterogeneous azeotropic distillation, and extractive distillation, and the like.

The hydrogenolysis reaction may have a selectivity for ethylene glycol may be 65% or greater (e.g., 65% to 95%, 70% to 95%, or 70% to 90%). The hydrogenolysis reaction may have a selectivity for propylene glycol may be 25% or less (e.g., 1% to 25%, or 1% to 20%).

*Example 1*. High-purity alpha-alumina powder from Nippon Light Metal was sieved to sized ranging from about 20 µm to about 38 µm. The sieved alpha-alumina powder was dried in a vacuum oven at about 100°C overnight. The dry weight was used to determine the weight of metal precursors. To achieve about 3.4 wt% nickel and about 0.9 wt% rhenium on the alpha-alumina particles, about 10.70 g nickel (II) nitrate hexahydrate and about 0.82 g ammonium perrhenate were added to about 37.56 g of deionized water. The catalyst precursor solution was dripped slowly onto about 60.83 g of the sieved alpha-alumina powder while constantly mixing the sieved alpha-alumina powder. The resulting material was spread in a thin layer and set to dry in a furnace at about 100°C overnight. Approximately 40 g of dried powder was broken up and added to a vertically oriented, 1-inch furnace tube. Gas was flown up through the powder, and a vibration motor was used to keep the material from forming slugs and preventing pressure buildup across the bed. The material was again dried for about 2 hours at about 120°C with gas (about 87 sccm nitrogen and about 13 sccm air) up flowing through the bed. Calcination was achieved by heating at about 2 °C/min up to about 500°C and holding for about 14 hours with the same gas flow of about 87 sccm nitrogen and about 13 sccm air. After cooling to about 150°C, the powder was purged with nitrogen for 30 minutes before changing to 100% H₂ flow. The reduction was a two-step reduction with a first step of heating to about 230°C over about 30 minutes and holding for about 7 hours and a second step of heating to about 340°C over about 40 minutes and holding for about 21 hours. The powder was then allowed to cool to room temperature. The tube was purged with nitrogen for about 30 minutes before introducing air. The catalyst was passivated with air and nitrogen for about 14 hours.

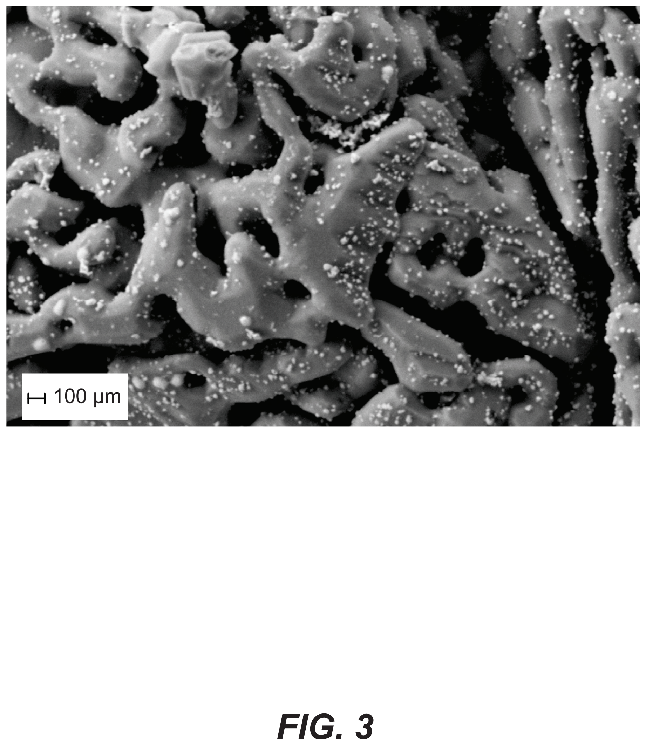

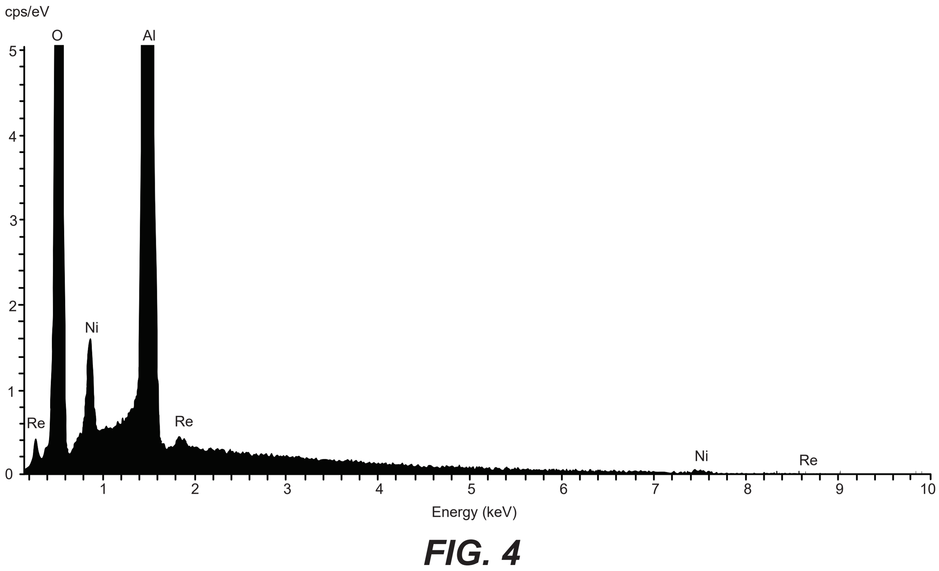

The resulting nickel-rhenium catalyst were analyzed using scanning electron microscopy (SEM) and energy dispersive x-ray spectroscopy (EDS). FIG. 3 is the SEM image of the resulting nickel-rhenium catalyst. FIG. 4 illustrates an EDS plot (counts per second per electron volt) showing the elemental composition of the nickel-rhenium catalyst. The plot shows that the nickel is at a higher concentration than the rhenium, which are both at significantly lower concentrations than the alumina (i.e., aluminum and oxygen). FIG. 3 and FIG. 4 demonstrate that the methods described herein produce catalysts characterized by small crystallite sizes, high surface area, and uniform metal distribution across the support material. Without wishing to be bound by theory, it is believed that the resulting nickel-rhenium catalyst provides an optimal combination of surface area and active site dispersion, which facilitates enhanced conversion of sugar to glycol over reduced reaction times.

*Example 2.* Hydrogenolysis reaction runs were conducted using a sugar-based reaction mixture under consistent reaction conditions, specifically a temperature of 245 °C and a hydrogen pressure of 1560 psia. A series of catalysts were evaluated in the presence of a tungsten co-catalyst. Examples 2D, 2E, 2F, and 2G employed nickel-rhenium catalysts with varying nickel to rhenium ratios and/or differing concentrations of the tungsten co-catalyst. Example 2A utilized a zirconia support in the absence of nickel and rhenium. Example 2B utilized a conventional Raney Ni catalyst without rhenium. Example 2C utilized a nickel-rhenium catalyst supported on theta-alumina.

Examples 2A-2F were conducted using a 1-liter continuous stirred tank reactor (CSTR) configured for high-pressure hydrogenation. The reactor body was constructed from stainless steel and fitted with a 2 µm internal filter to retain solid catalyst particles. The system included a heating mantle and insulation jacket to maintain reaction temperature, and was sealed with a graphite gasket. Hydrogenation catalyst was loaded directly into the reactor prior to sealing and leak testing the CSTR reactor. The catalyst was dispersed in a flushing solution (30/70 v/v ethylene glycol/water), which also served as the initial reaction medium.

The sugar feed used in Examples 2A-2F consisted of aqueous glucose solution and a tungsten salt introduced at a controlled rate, 3 sccm. The glucose concentration for the examples was 30-32 wt% glucose. Hydrogen gas was supplied to the reactor at a constant rate, 0.7 SLPM. The CSTR was mixed at a constant stirring speed, 500 rpm. Conversion was monitored using off-line sample analysis, namely gas chromatography (GC) and high pressure liquid chromatography (HPLC). Reaction time was based on time since the sugar feed was initiated. Effluent samples were taken every 3-4 hours. GC and HPLC data were used to quantify glycol yield and selectivity.

After each run in Examples 2A-2F, the reactor was flushed, depressurized, and opened for inspection. Catalyst was recovered, dried, and weighed to assess loss and degradation. Reactor internals were cleaned, and filters replaced to ensure consistency across runs.

**Table 1**

| Ex. | Ni (wt.%) | Re (wt.%) | Support | [W] (ppm) | Temp. (°C) | Pres. (psia) | Reaction Time (hrs) | MEG Yield (%) |
|---|---|---|---|---|---|---|---|---|
| 2A | - | - | Zirconia | 3500 | 245 | 1560 | 4 | 11.6 |
| 2B | 99.00 | - | Raney Ni | 3500 | 245 | 1560 | 20 | 41.6 |
| 2C | 6.80 | 1.80 | θ-alumina | 3500 | 245 | 1560 | 9 | 65.9 |
| 2D | 6.80 | 1.80 | α-alumina | 3500 | 245 | 1560 | 7 | 71.5 |
| 2E | 6.80 | 1.80 | α-alumina | 2310 | 245 | 1560 | 4 | 76.5 |
| 2F | 10.20 | 3.60 | α-alumina | 3500 | 245 | 1560 | 26 | 80.5 |
| 2G | 3.40 | 0.90 | α-alumina | 1750 | 245 | 1560 | 21 | 79.8 |

Hydrogenolysis reactions conducted using nickel-rhenium bimetallic catalysts supported on alpha-alumina particles demonstrated superior performance relative to a standard Raney nickel catalyst. Examples 2D, 2E, 2F, and 2G exhibited significantly higher monoethylene glycol conversions, ranging from 71.5% to 80.5%, whereas Example 2B (Raney Ni) yielded a lower conversion of 41.6%.

Hydrogenolysis reactions conducted using nickel-rhenium bimetallic catalysts supported on alpha-alumina particles unexpectedly demonstrated superior performance relative to the same metals supported on theta-alumina particles, despite theta-alumina generally exhibiting a higher surface area. Example 2D (nickel-rhenium on alpha-alumina) achieved a monoethylene glycol yield of 71.5%, whereas Example 2C (nickel-rhenium on theta-alumina) exhibited lower performance under comparable conditions. Nickel-rhenium catalysts having a total weight ratio of nickel to rhenium ranging from 3.5:1 to 4:1 were found to be particularly effective.

Hydrogenolysis reactions conducted using nickel-rhenium catalysts supported on alpha-alumina particles, in combination with tungsten co-catalyst concentrations below 3500 ppm, demonstrated improved conversion efficiency, yielding higher percentages of monoethylene glycol. Example 2E (2310 ppm W) and Example 2G (1750 ppm W) achieved monoethylene glycol conversions of 76.5% and 79.8%, respectively, which were notably higher than the conversion observed in Example 2D (3500 ppm W).

The foregoing description of certain examples, including illustrated examples, has been presented only for the purpose of illustration and description and is not intended to be exhaustive or to limit the disclosure to the precise forms disclosed. Numerous modifications, adaptations, and uses thereof will be apparent to those skilled in the art without departing from the scope of the disclosure. For instance, any examples described herein can be combined with any other examples to yield further examples.

## Claims

1. A method comprising:
impregnating alpha-alumina particles with catalyst precursors comprising a nickel-containing salt and a rhenium-containing salt to produce precursor-impregnated alpha-alumina particles;
drying of the precursor-impregnated alpha-alumina particles in a fluidized bed at a first temperature; and
calcining then reducing then passivating the precursor-impregnated alpha-alumina particles in the fluidized bed to produce a nickel-rhenium catalyst comprising a nickel-rhenium mixed metal oxide or alloy on the alpha-alumina particles.

2. The method of claim 1, wherein the nickel-rhenium catalyst has a total weight ratio of nickel to rhenium ranging from 2:1 to 10:1, optionally ranging from 3.5:1 to 4:1.

3. The method of any preceding claim, wherein the nickel-rhenium catalyst comprises 1 wt% to 99 wt% of the nickel-rhenium mixed metal oxide or alloy, based on a total weight of nickel and rhenium.

4. The method of any preceding claim, wherein the nickel-rhenium catalyst has a weight ratio of alumina to nickel and rhenium, cumulatively, ranging from 50:1 to 99.5:1.

5. The method of any preceding claim, wherein impregnating of the alpha-alumina particles comprises contacting the alpha-alumina particles and a precursor solution that comprises the catalyst precursors and water.

6. The method of claim 5, further comprising continuously agitating the alpha-alumina particles while contacting the alpha-alumina particles with the precursor solution.

7. The method of any preceding claim further comprising:
drying the precursor-impregnated alpha-alumina particles at a second temperature before drying at the first temperature, wherein the first temperature is greater than the second temperature.

8. The method of claim 7, wherein the drying at the second temperature is not in a fluidized bed.

9. The method of any preceding claim, wherein the reducing step is a two-stage reducing step, wherein a first stage is performed at a temperature ranging from 200°C to 300°C and for a time ranging from 3 hours to 12 hours, and wherein a second stage is performed at a temperature ranging from 275°C to 400°C and for a time ranging from 12 hours to 36 hours.

10. A method comprising:
producing a reaction mixture comprising a sugar, a nickel-rhenium catalyst, a tungsten co-catalyst, and a solvent to produce a reaction mixture, wherein the nickel-rhenium catalyst comprises a nickel-rhenium mixed metal oxide or alloy on alpha-alumina particles; and
contacting the reaction mixture and hydrogen at conditions suitable for hydrogenolysis of the sugar to glycol.

11. The method of claim 10, wherein the nickel-rhenium catalyst has a total weight ratio of nickel to rhenium ranging from 2:1 to 10:1, optionally ranging from 3.5:1 to 4:1.

12. The method of any one of claims 10-11, wherein the nickel-rhenium catalyst comprises 1 wt% to 99 wt% of the nickel-rhenium mixed metal oxide or alloy, based on a total weight of nickel and rhenium.

13. The method of any one of claims 10-12, wherein the nickel-rhenium catalyst has a weight ratio of alumina to nickel and rhenium, cumulatively, ranging from 50:1 to 99.5:1.

14. The method of any one of claims 10-13, wherein the reaction mixture comprises 10 wt% to 60 wt% of the sugar.

15. A nickel-rhenium catalyst comprising a nickel-rhenium mixed metal oxide or alloy impregnated on alpha-alumina particles.
